# EUROPÄISCHE PATENTANMELDUNG

(11) **EP 2 090 720 A2**
(43) Veröffentlichungstag der Anmeldung: **19.08.2009**
(21) Anmeldenummer: 09152233.4
(22) Anmeldetag: 06.02.2009
(51) Int. Cl.: E04H 1/02

(54) **Pyramidenförmiges Gebäude**

(30) Priorität: 18.02.2008 AT 22632008
(71) Anmelder: Holzweber, Maria Elisabeth, 9523 Landskron (AT); Wasserfaller, Georg jr., 9523 Landskron (AT)
(72) Erfinder: Wasserfaller sen., Georg, 9523, Landskron (AT)
(74) Vertreter: Cremer, Christian

(57) **Zusammenfassung**

Die vorliegende Erfindung bezieht sich auf ein pyramidenförmiges Gebäude, mit einem vierseitigen Basiskörper aus Holz, wie Nadelholz.

Indisponierte bzw. kranke, insbesondere gestresste bzw. verspannte Menschen haben einen Bedarf an Aufenthaltsorten, an denen Sie Kraft tanken und Entspannung und Erholung finden können. Wenn Aufenthaltsorte Linderung oder sogar, zumindest teilweise, Heilung beim Auftreten von Krankheiten wie Leukämie bieten können, dann kann mit dem Anbieten besonderer Aufenthaltsorte ein ganz besonders nachgefragter Bedarf gedeckt werden.

Durch zahlreiche Untersuchungen und Versuche hat der Erfinder der vorliegenden Erfindung erkannt, dass ein pyramidenförmiges Gebäude aus zahlreichen Gründen zur Deckung des Bedarfs beitragen kann.

## Beschreibung

Die vorliegende Erfindung bezieht sich auf ein pyramidenförmiges Gebäude, mit einem vierseitigen Basiskörper aus Holz, wie Nadelholz.

### Stand der Technik

Pyramidenförmige Gebäude sind seit alters her bekannt. Beispielsweise sind die Grabstätten vieler ägyptischer Pharaonen des Alten Reiches aus der Zeit von ca. 2680 bis 2180 vor unserer Zeitrechnung pyramidenförmige Bauten. Als Beispiel kann die Cheopspyramide genannt werden. Bei diesen Bauten handelt es sich meist um Stufenpyramiden, die aus Stein, insbesondere aus großen Steinblöcken, erbaut wurden. Als Baumaterial wurden zu Blöcken verarbeitete Felsbrocken von weichem Gestein aus Felswänden herausgebrochen. Die Außenseiten waren ursprünglich mit weißem Kalkstein verkleidet. Für den Kernbau wurde jedoch grober Nummulitenkalkstein von minderer Qualität verwendet. Aus hartem Tiefengestein, wie Rosengranit, wurden Sarkophagkammern, Verkleidungsblöcke, Scheintüren, Blockierungsblöcke, Verstärkungen des Korridors, das Gangsystem der Innenräume, Pfeiler, Säulen und Türkonstruktionen der Pyramide gefertigt. Für Pyramidenböden wurde Kalzit von besonders guter Qualität verwendet. Für die Böden der Cheops-Pyramide wurde das sehr harte und schwarze vulkanische Gestein Basalt verwendet.

Die spärlichen Holzressourcen Ägyptens, vorwiegend Dattelpalmen-, Akazien-, Dumpelpalmen-, und Tamariskenholz, waren für so große Bauten wie die Pyramiden ungeeignet. Funde aus Gräbern der 1. Dynastie belegen, dass aus dem syrischpalästinensischen Raum importierte Nadelhölzer hauptsächlich zur Herstellung von Schlitten, Rollen, Hebeln und Hämmern, d. h. von Werkzeugen zur Herstellung einer Pyramide, verwendet wurden.

Aus der DE 25 03 622 A1 ist eine Energieabsorptionskammer, insbesondere in Form einer viereckigen Pyramide, mit gleichseitigen Seitenwänden, insbesondere aus Holz, mit einer Basis und einem Gestell im Inneren der Pyramide und mit reflektierenden, insbesondere spiegelnden Innenwänden bekannt. Nachteilig an dieser Pyramide ist, dass durch die Verwendung von Spiegeln bei sonstiger Bauweise aus Holz ein inhomogenes Bauwerk entsteht.

Die DE 20 2006 004 778 U1 beschreibt eine ähnlich der Cheopspyramide geformte Pyramide mit einer energieverstärkenden Fokussiereinrichtung, die aus Fokussierungstäben gebildet ist, die als Halterung für eine Energie-Plattform dienen und gleichzeitig Energie zu einem Energetisierungsobjekt fokussieren sollen. Nachteilig an dieser Pyramide sind ihr offener Aufbau einerseits und die Verwendung von Metallstäben als Baumaterial andererseits.

Aus der RU 2 175 254 C1 ist eine Vorrichtung zur Energiekorrektur in Form einer vierflächigen Pyramide aus einem dielektrischen Material mit einer Innenkammer bekannt. Die Vorrichtung besteht aus zwei Teilen, von denen der untere die als hohler Pyramidenstumpf hergestellte Basiskammer und der obere ein Quarz-Endstück darstellt. Weiterhin sind eine spiralförmige Antenne in dem Pyramidenstumpf und eine innere Resonanz-Einheit entlang der vertikalen Achse der Pyramide angeordnet. Die Facetten des Pyramidenstumpfes können wahlweise aus Holz hergestellt sein. Sowohl die Antenne als auch die innere Resonanz-Einheit der Korrektorvorrichtung sind vorzugsweise aus Metall hergestellt. Ein Auflageteil ist wahlweise als Tischoberfläche ausgebildet. Die externen und internen Dimensionen der Pyramide und der Antenne und der inneren Resonanz-Einheit in ihrem Inneren sind so gewählt, dass sie in Größenverhältnissen des goldenen Schnitts vorliegen. Nachteilig an dieser Pyramide sind wieder die Vermengung von unterschiedlichen Materialien wie Holz und Metall.

Die DE 297 06 928 U1 beschreibt ein Pyramidenmodell mit 40 cm Basisbreite mit einer Bergkristall-Pyramide in der Apix, die auf einer eingeschweißten Edelstahlplatte steht, wobei die Seiten der Pyramide mit einer Kappe aus Edelstahl verkleidet sind und an den Innenseiten der Pyramidenkanten ein abgesägtes Edelstahlgestänge angeschweißt ist. An den vier Seiten der Pyramidenverkleidung sind Buchsen für Bananenstecker angebracht, die im rechten Winkel zur schrägen Pyramidenfläche gebohrt sind. Die in die Buchsen eingeführten Bananenstecker müssen die Bergkristall-Pyramide berühren. Nachteilig an dieser Pyramide sind wieder deren inhomogener Aufbau aus Stahl und der Umstand, dass sich Menschen in ihr nicht aufhalten können.

Aus der DE 87 10 871 U1 ist eine Hohlpyramide, insbesondere mit quadratischer offener Grundfläche, bekannt, die aus Materialien aufgebaut ist, die bestimmte Metalle bzw. Kunststoff enthalten bzw. aus diesen bestehen. Nachteilig an dieser Pyramide ist wieder deren inhomogener Aufbau aus Materialien wie Metallen und Kunststoff.

Die DE 36 30 523 A1 beschreibt eine der Abnahme von Pyramiden-Energie für medizinische, biologische und technische Zwecke dienende Pyramiden-Energie-Abnahmevorrichtung. Die Abnahmevorrichtung ist außerhalb der Seitenflächen der Pyramide angeordnet, wobei sie zur Abnahme von Pyramiden-Energie eine ableitende um den Pyramidenkörper geschlungene, insbesondere spiralförmig geschlungene Leitung aufweist, die so weitergeführt ist, dass ihre Windungen und Weiterführung z. B. über Bänder, Seile , eine Halterung, einen Stützring oder einen Innenstützring den gesamten Pyramidenkörper überstreicht. Der Pyramidenkörper ist mit parallel liegenden Grundkanten in Nord-Süd-Ausrichtung ausgerichtet. Auch hier sind wieder der inhomogene Aufbau der Pyramide und insbesondere die außerhalb der Pyramide angeordnete Ableitvorrichtung nachteilig.

Die NZ 299 753 offenbart eine Minigesundheitspyramide aus Holz mit einem Kristall in ihrer Basis und im Schlussstein. Nachteilig an dieser Pyramide ist, dass sie so klein ausgebildet ist, dass sich Menschen in ihr nicht aufhalten können und auch dass sie inhomogen d. h. aus unterschiedlichen Materialien aufgebaut ist.

Aus der RU 22 59 188 ist eine Phytosauna in Form einer als dreiseitiges Prisma aus Holz ausgebildeten Pyramide bekannt. Menschen können sich zur Behandlung darin aufhalten. Die Pyramide wird von Dampfströmen durchströmt, die zur Entspannung der sich in ihr aufhaltenden Menschen dienen sollen.

In der DE 10 240 296 A1 sind der Aufbau und die Wirkungen eines pyramidenförmigen Gerätes, vorzugsweise ohne Basiskörper und auf die Spitze gestellt, mit vier Seitenwänden, beispielsweise aus Holz, die zumindest an den Innenflächen mit mindestens einer Metallsorte zumindest teilweise verkleidet ist, beschrieben. Nachteilig sind die vorzugsweise Anordnung "Spitze unten" und "Basisseite oben" sowie der vorzugsweise fehlende Basiskörper, sowie der wiederum inhomogene Aufbau aus unterschiedlichen Materialien wie Holz und Metall.

Die DE 38 06 503 A1 beschreibt eine Strahlenschutz-Pyramide aus Chromnickel, Chromnickelgewebe mit einer bestimmten Maschenweite bzw. aus Spiegelglas zur Erzielung einer erdstrahlenfreien Schlaf- und Wohnfläche durch Reflexion der Strahlen. Auch bei dieser Pyramide ist deren Aufbau aus Materialien, die kein Holz sind, nachteilig.

Aus der WO 01/32252 A1 sind mehrere Gegenstände bekannt, nämlich (1) eine verschweißte, doppelwandige Pyramide mit eingesetztem Einsatzteil und (2) eine einschalige Pyramide mit einem zweigeteilten Becken und einer überbauten Metallkonstruktion. Die in der WO 01/32252 A1 beschriebene Pyramide überdacht eine Art Badewanne, in die unter anderem eine Salzlösung eingelassen werden kann. Die Pyramide besteht aus Metall oder aus Stein. Es ist eine Vorrichtung zur Regulierung des Sauerstoffgehaltes im Inneren und auch eine Belüftung vorhanden.

Aus der DE 27 09 552 A1 ist eine Pyramide als dreistöckiges Wohngebäude bekannt, das aus einem Metallrahmen und darin aufgehangenen Holzwänden besteht. Das Gebäude hat in jeder Etage eine Terrasse oder einen Balkon und darauf hinausführende Türen. Auch in der Beschreibung der DE 27 09 552 A1 wird die vitalitätsfördernde Eigenschaft der Gebäudeform dargelegt.

Der Gedanke, Pyramiden für den Aufenthalt von Menschen zu bestimmen, wird in den letzten Jahrzehnten gem. der Patentliteratur immer wieder aufgegriffen. Hierbei lehrt die Literatur, dass elektromagnetische Strahlungen, sonstige ionisierende Ströme und Strahlungen und externe Einflüsse mittels metallischer Maßnahmen abzufangen seien. Auf jeden Fall herrscht die Meinung vor, dass metallische Verbindungstechniken keinen negativen Einfluss auf das Raumklima einer begehbaren Pyramide haben sollten.

Weiterhin ist es aus elektronischen Beschreibungen bekannt, dass Lärchenholz zur Ausbildung einer weitgehend offen ausgebildeten mehr hohen als breiten so genannten "Kürbispyramide" verwendet werden kann. Eine ebenfalls weitgehend offen ausgebildete, eher spitze Pyramide aus Nadelholz, nämlich aus Fichtenholz, ist den elektronischen Beschreibungen ebenfalls zu entnehmen. Eine Abbildung einer Pyramide aus Holz wurde auch zum Anmeldezeitpunkt unter http://www.energiaspiral.hu/nemet.html aufgefunden. Eine Abbildung einer Gartenpyramide aus Holz wurde zum Anmeldezeitpunkt unter http://www.pyramida.sk/eng/produkty/proden.htm ganz unten auf der Seite aufgefunden. Bei diesen Pyramiden ist entweder die weitgehend offene Ausbildung oder ihre mehr hohe als breite Ausbildung nachteilig. Weiterhin ist es zweifelhaft, ob diese Pyramiden im Aufbau homogen ausgebildet sind.

### Erfindungsbeschreibung

Indisponierte bzw. kranke, insbesondere gestresste bzw. verspannte Menschen haben einen Bedarf an Aufenthaltsorten, an denen Sie Kraft tanken und Entspannung und Erholung finden können. Wenn Aufenthaltsorte Linderung oder sogar, zumindest teilweise, Heilung beim Auftreten von Krankheiten wie Leukämie bieten können, dann kann mit dem Anbieten besonderer Aufenthaltsorte ein ganz besonders nachgefragter Bedarf gedeckt werden. Die Befriedigung dieses Bedarfs gelingt mit einem pyramidenförmigen Gebäude gemäß Anspruch 1. Eine vorteilhafte Verwendung wird in Anspruch 15 gelehrt. Vorteilhafte Ausbildungen der Erfindung sind in den Ansprüchen 2 bis 14 definiert.

Bei dem pyramidenförmigen Gebäude mit einem vierseitigen Basiskörper aus Holz, wie Nadelholz, sind alle sich über den Basiskörper aufspannenden Seitenflächen erfindungsgemäß aus einer einzigen Holzart gefertigt. Der Basiskörper ist eine vom Boden abgehobene Fläche, die in der Regel bodennah angeordnet ist. Die Fertigung ist derart, dass eine Verbindung eines ersten und eines zweiten Holzbauteiles metallfrei zusammengefügt ist. Die Verbindung einzelner Holzbauteile miteinander und untereinander erfolgt durch nicht metallische Verbindungsarten, die teilweise aus anderen technischen Bereichen, nicht jedoch aus dem Bereich des Pyramidenbaus, bekannt sind. Diese homogene Ausbildung des erfindungsgemäßen Bauwerkes trägt entscheidend zur Ausbildung eines angenehmen, für manche Menschen deren Heilung unterstützenden bzw. sogar ermöglichenden Aufenthaltsklimas im Inneren des Bauwerkes bei.

Eine bevorzugte Weiterbildung des Gebäudes besteht darin, dass das Holz, insbesondere der tragenden Holzbauteile, eine Lärchenholzart ist. Es sind aus verschiedenen Regionen der Welt unterschiedliche Lärchenarten bekannt. Das Gebäude besteht aus wenigstens einer solchen Lärchenart, insbesondere aus einer in Europa fällbaren Lärchenart. Vorzugsweise sind sämtliche Verbindungen der Holzbauteile durch formschlussbildende, materialgleiche Mittel, wie Holzschraube, Holzdübel oder Holznagel, realisiert. Durch die ausschließliche Verwendung von Holz, insbesondere von einer Lärchenholzart, lässt sich ein besonders günstiges Aufenthaltsklima für Entspannung bzw. Linderung von Leiden suchende Menschen im Inneren bzw. zumindest in einem Teil des Innenraums der Pyramide ausbilden. Lärchenholzarten sind sehr harzreich, wenig dem Werfen und nicht dem Wurmfraß unterworfen und daher generell ausgezeichnet, als dauerhaftes Bauholz für Land- und Wasserbauten etc. geeignet.

Das pyramidenförmige Gebäude weist eine vom Basiskörper abgehobene, zentrisch zu einem Mittelpunkt des Basiskörpers angeordnete Aufenthaltsplattform auf. Der Abstand zwischen einer Oberfläche des Basiskörpers und einer Oberfläche der Aufenthaltsplattform ist geringfügig größer als ein Drittel einer Gesamthöhe des pyramidenförmigen Gebäudes. Durch die Anhebung der Aufenthaltsplattform auf die beschriebene Höhe können sich Menschen in der im Bezug auf die Entspannung wirksamsten bzw. die Gesundheit besonders unterstützenden Zone in der Pyramide aufhalten.

Vorzugsweise weist das pyramidenförmige Gebäude einen einzigen zusammenhängenden Innenraum auf. Dadurch kann im Hinblick auf die Entspannung bzw. die Gesundheitsförderung eine störungsfreie und besonders intensive Wirkung erzielt werden.

Es ist bevorzugt, wenn das pyramidenförmige Gebäude eine geschlossene Außenhülle, vorzugsweise mit einer Schindelbedeckung, aufweist. Durch die geschlossene Außenhülle kann der Innenraum von der Außenwelt abgeschlossen werden. Die Schindelbedeckung ergibt eine wetterfeste, insbesondere wasser- und windundurchlässige Außenhülle. Es ist bekannt, dass Schindeln aus einer Lärchenholzart chemisch unbehandelt eine Lebensdauer von rund 100 Jahren oder mehr haben können.

Es ist besonders bevorzugt, wenn die unbehandelten Schindeln jeweils mindestens eine Durchgangsöffnung aufweisen, in die ein mit einer Seitenfläche verbundener Dorn ragt. Dadurch lassen sich vorteilhafterweise die Schindeln an dem gewünschten Ort an der Außenhülle befestigen, ohne dass ein unerwünschtes Verrutschen zu befürchten wäre. Besonders bevorzugt ist es, wenn sich die Schindeln in der Art von Fischschuppen überlappen und so durch Auflagereibung gegenseitig an einer Pyramidenfläche festhalten.

Besonders vorteilhaft ist es weiterhin, wenn die Seitenflächen zumindest doppelwandig mit einem zwischen ihren mindestens zwei Wänden angeordneten Dämmraum ausgebildet sind, der vorzugsweise mit Schafswolle ausgefüllt ist. Auf diese Weise lässt sich die ohnehin wegen der Verwendung des Holzes bereits sehr gute Wärmedämmfähigkeit der Pyramidenwände weiter steigern, wobei durch die Verwendung der Schafswolle, das natürliche wohltuende Klima in der Pyramide nicht nur nicht beeinträchtigt, sondern sogar noch gesteigert werden kann.

Besonders bevorzugt ist es auch, wenn die Seitenflächen jeweils nach einer der vier Haupthimmelsrichtungen ausgerichtet sind. Es ist beobachtet worden, dass durch die Ausrichtung die Pyramide in ihrem Inneren die oben beschriebene entspannende bzw. gesundheitsfördernde Wirkung in besonderem Maße entwickeln kann.

Weiterhin ist es bevorzugt, wenn das Flächenverhältnis der Aufenthaltsplattform zum Basiskörper mindestens etwa ein Zehntel (mit einer Abweichung von ca. 10 % gegenüber der größeren der beiden Flächen) beträgt. Dieser Wert ist aus Überlegungen und Versuchen des Erfinders hervorgegangen und entspricht somit einem günstigen Erfahrungswert.

Vorteilhafterweise stellt ein Belüftungssystem die Klimatisierung des pyramidenförmigen Gebäudes bereit. Durch das Belüftungssystem kann in der heißen Jahreszeit kühle und in der kalten Jahreszeit warme Luft in das Innere der Pyramide geleitet werden, was zu einer Steigerung des Wohlbefindens der sich in der Pyramide, insbesondere auf der Aufenthaltsplattform, aufhaltenden Menschen beiträgt. Die Lüftungskanäle des Belüftungssystems können auch verwendet werden, um Luft aus dem Inneren der Pyramide abzusaugen. Abgesaugte Luft kann ins Freie d. h. in die Umgebung der Pyramide verbracht werden. Es ist auch möglich, Luft vor ihrer Einleitung bzw. nach ihrer Absaugung mittels des Belüftungssystems einer Aufbereitungsanlage zuzuführen. Eine solche Aufbereitungsanlage kann in einem separaten Gebäude in der Nähe der Pyramide untergebracht sein. Durch einen den Jahreszeiten bzw. Behandlungserfordernissen angepassten Betrieb des Belüftungssystems werden auch die Luftströmungsverhältnisse in der Pyramide überaus günstig beeinflussbar. Die Belüftung erfolgt durch ein auf wenigstens zwei Ebenen innerhalb der Pyramide angebrachtes Lüftungskanalsystem mit entsprechenden Lüftungsschlitzen. Einer der Kanäle, vorzugsweise der obere beim Beheizen, ist für Zuluft zuständig, während der andere, vorzugsweise der untere beim Beheizen, für die Entsorgung der verbrauchten Luft zuständig ist. Somit erfolgt eine permanente Luftschichtumwalzung in der Pyramide. Das Lüftungskanalsystem hat wenigstens zwei Lüftungskanäle auf unterschiedlichen Höhen innerhalb des pyramidenförmigen Gebäudes, von denen somit nur ein Lüftungskanal, insbesondere der obere im Falle eines Beheizens, ein Zuluftkanal ist. Der Belüftungskanal kann, insbesondere in den warmen bzw. heißen Monaten des Jahres, auf Kühlbetrieb umgeschaltet werden. Die Umschaltung kann beispielsweise von einem Nebengebäude oder von einer unterirdischen Infrastruktur aus erfolgen. Beim Kühlen wird die Zuluft vorzugsweise durch den oberen, vorzugsweise horizontalen, Lüftungskanal zugeführt. Die Abluft wird vorzugsweise durch den unteren, vorzugsweise horizontalen, Lüftungskanal abgeführt.

Besonders vorteilhaft ist eine Ausbildung, bei der die Aufenthaltsplattform kreuzförmig ausgebildet ist. Die vier Aussparungen der kreuzförmigen Aufenthaltsplattform jeweils an zwei entlang eines Schrägsparrens miteinander verbundenden Seitenflächen bilden dann nämlich eine die Klimatisierung unterstützende Strömungsecke. Durch diese forminduzierte weitere Strömungsleitung und -lenkung ergibt sich in der Pyramide ein stabiles stationäres Luftströmungsfeld, das die positive Wirkung, insbesondere durch eine besondere Wohlfühlatmosphäre, der Pyramide unterstützt.

Ganz besonders bevorzugt ist es, wenn das pyramidenförmige Gebäude, vorzugsweise mit Ausnahme eines zumindest weitgehend in seinem Inneren in Luft persistierenden Stromes Ionen, gänzlich frei von Vorrichtungen ist, die einen elektrischen Strom oder einen Wasserstrom leiten können. Solche Vorrichtungen sind Wasserleitungen aus Kupfer oder Stahl und elektrische Kabel aus Kupfer oder Aluminium. Der persistierende Strom kann durch natürliche Konvektion der Luft innerhalb der Pyramide entstehen. Mit anderen Worten, das Gebäude weist in seinem Inneren - mit Ausnahme der sich in ihr aufhaltenden Menschen - nur Gegenstände bzw. Materialien auf, die gemeinhin als "Nicht-Leiter" bekannt sind. Durch die praktisch völlige Freiheit von insbesondere leitungsgebundenen elektrischen Strömen bzw. Wasserströmen kann sich die positive Wirkung der Pyramide ungestört von solchen Strömen und deren Wirkungen entfalten.

Die Konstruktion bzw. den Aufbau des Gebäudes betreffend ist es vorteilhaft, wenn die tragenden Holzbauteile Sparren, insbesondere Schrägsparren, sind. Günstigerweise ist zumindest an dem einen Ende von bestimmten solcher Holzbauteile ein ausgeklinkter Schwalbenschwanz auf Verzug angeordnet. Dadurch kann eine feste, sichere und dauerhafte Verbindung des ersten und des zweiten Holzbauteiles metallfrei zusammengefügt werden.

Weiterhin ist es vorteilhaft, wenn auf der Aufenthaltsplattform lehnenlose Bänke, insbesondere vier Holzbänke angeordnet sind. Die Bänke sind vorzugsweise parallel zu wenigstens einer Seitenfläche des Basiskörpers ausgerichtet. Durch die Bänke können die Menschen eine Haltung auf der Aufenthaltsplattform einnehmen, die für die Entfaltung der von der Pyramide ermöglichten Wirkung, besonders günstig ist.

Für spezielle Fälle ist es vorteilhaft, eine außermittig befestigte Umlenkrolle für ein Tragemittel, wie ein Seil, insbesondere ein Hanfseil vorzusehen. Diese in der Nähe der Pyramidenspitze angeordnete durch ihre in Bezug auf die Symmetriezentralachse der Pyramide außermittige Befestigung das Seil exakt in die der vertikalen Symmetriezentralachse der Pyramide entsprechenden Richtung lenkende Umlenkrolle ermöglicht die pendelhafte Aufhängung eines insbesondere sechs-flächigen Kristalls, vorzugsweise eines naturbelassenen, unbearbeiteten Bergkristalls. Der Kristall konzentriert die Wirkung der lonenströme, insbesondere des negativen lonenstromes, auf einen unter dem Kristall sich aufhaltenden Menschen. Insbesondere zur gezielten lokalen Applikation der lonenwirkung (siehe nächster Absatz) auf einen Körper bzw. auf einen Körperbereich bzw. auf ein Organ, insbesondere des menschlichen Körpers, ist der Kristall vorteilhaft. Der Kristall stört die Homogenität des Bauwerkes nicht.

Ganz besonders vorteilhaft ist es, wenn ein sich zumindest weitgehend in Luft bewegender Strom von Ionen zumindest im Inneren des pyramidenförmigen Gebäudes persistiert.

Insbesondere ist es bevorzugt wenn der Ionenstrom, einen größeren Anteil an negativen als an positiven Ionen aufweist, beispielsweise im Verhältnis 80:20.Die in Luft persistierenden Ströme von Ionen sind unabdingbar erforderlich, damit die Pyramide ihre insbesondere der Gesundheit zuträglichen Wirkung entfalten kann. Der positive und der negative Ionenstrom unterstützen insbesondere in ihrem Zusammenwirken die Selbstheilungs- und Selbstregenerationskräfte des menschlichen Körpers. Beispielsweise ist schon beobachtet worden, dass ein solcher Ionenstrom günstig auf die Hämoglobinbildung bzw. auf die Bildung roter Blutkörperchen im menschlichen Körper wirken kann, was wiederum bei der Behandlung von Leukämie vorteilhaft sein kann. Die lonenströme werden durch den erfindungsgemäßen insbesondere homogenen Aufbau des Gebäudes auf eine besonders natürliche Art und Weise auf einen Körper aufbringbar bzw. in einen Körper einbringbar. Es wird berichtet, dass beispielsweise sogar bestimmten Formen der Leukämie und anderen Formen von Krebserkrankungen durch den wiederholten Aufenthalt von Erkrankten in einem negative und positive Ionen transportierenden Luftstrom vorteilhaft entgegengewirkt werden kann oder diese zumindest gelindert werden konnten.

Vorteilhafterweise besitzt der Basiskörper eine quadratische Grundfläche, vorzugsweise mit einer Seitenlänge von zwölf mal zwölf Metern. Diese Größe hat sich erfahrungsgemäß als ideal für eine eine Gruppe von etwa 6 bis 12 Menschen auf ihrer Aufenthaltsplattform fassende Pyramide erwiesen.

Besonders vorteilhaft ist eine Höhe des pyramidenförmigen Gebäudes, die in einer durch einen goldenen Schnitt bestimmbaren Relation zur Seitenlänge des Basiskörpers steht. Insbesondere ist bei zwölf Metern Seitenlänge eine Höhe von 7,68 Meter besonders vorteilhaft. Diese Werte sind durch praktische Versuche bestimmte Erfahrungswerte und ergeben eine vorteilhafte Größe der Pyramide und insbesondere jenes Bereiches auf der Aufenthaltsplattform, in dem sich die Menschen bestimmungsgemäß aufhalten.

Weiterhin ist es günstig, wenn die Verbindung eines ersten und eines zweiten Holzbauteiles mittels eines Leimes, vorzugsweise auf Holzbasis oder auf Holzharzbasis, insbesondere auf der Basis eines Harzes einer Lärchenholzart, in ihrer Haltefähigkeit zumindest unterstützt wird. Durch die Verwendung eines solchen Leimes kann auf andere insbesondere industriell bzw. nicht naturnah hergestellte Kleber verzichtet und die natürliche die Gesundheit fördernde Bauweise der Pyramide unterstützt werden.

Weiterhin ist es bevorzugt, wenn ein splittbefüllter Bereich, insbesondere für den Wasserabfluss, zumindest unterhalb des Basiskörpers vorgesehen ist. Durch den splittbefüllten Bereich, man könnte auch von einem Splittuntergrund sprechen, kann Wasser, insbesondere Regenwasser, schneller und vollständiger in den umgebenden Untergrund versickern. Durch den Splittuntergrund kann somit verhindert werden, dass das Holz, insbesondere des Basiskörpers längere Zeit im Wasser liegt. Als Material ist Straßensplitt bevorzugt, insbesondere mit einer Korngröße 6-8 mm. Als Nahbereich wird ein Umkreis von ca. 1 m um die Pyramide betrachtet. Nach einer alternativen Ausgestaltung kann auch nur der Bereich unmittelbar vor der Pyramide mit Splitt aufgefüllt sein.

Bevorzugt ist es weiterhin, wenn in einen Untergrund des Basiskörpers eingelassene, insbesondere dort verankerte, Pflöcke vorhanden sind. Die Pflöcke dienen insbesondere zur Verankerung des pyramidenförmigen Gebäudes gegen seitliches Abgleiten, beispielsweise in Erdbebengebieten oder auf schrägen Liegenschaften.

Besonders bevorzugt ist es, wenn sich der Aufstellungsort des pyramidenförmigen Gebäudes an einem positiven Kraftpunkt befindet. In diesem Zusammenhang ist unter einem positiven Kraftpunkt ein Ort zu verstehen, an dem im Vergleich mit anderen erdnahen Orten ein vermehrtes Vorkommen von Ionen, insbesondere von negativen Ionen, feststellbar ist. Die Ionen sind beispielsweise mit einem Luftionenmessgerät feststellbar. "Positiv" wird der Kraftpunkt nicht etwa seiner positiven elektrischen Ladung wegen genannt, sondern wegen der positiven Wirkung, die ein solcher positiver Kraftpunkt auf Menschen haben kann, die sich in unmittelbarer Nähe oder direkt im Zentrum eines solchen aufhalten.

Der Innenraum des pyramidenförmigen Gebäudes ist zusammenhängend ausgebildet. Das Merkmal des Zusammenhangs des Innenraums kann topologisch betrachtet als wegzusammenhängend bezeichnet werden. Der Zusammenhang besteht global. Der Zusammenhang besteht auch lokal. Alternativ zu wegzusammenhängend können die Bezeichnungen pfad-zusammenhängend oder kurvenweise zusammenhängend verwendet werden. Durch den Zusammenhang kann sich im Innenraum vorteilhafterweise eine, vorzugsweise symmetrische, Strömung, wie Luftströmung, ausbilden. Die Strömung kann global im Innenraum persistieren. Die Strömung kann auch lokal in dem Innenraum persistieren.

Die geschlossene Außenhülle des pyramidenförmigen Gebäudes wird durch die an den Schrägsparren paarweise miteinander verbundenen Seitenflächen und den Basiskörper gebildet. Im Bereich der Seitenflächen kann die geschlossene Außenhülle durch eine Schindelbedeckung realisiert sein. Die geschlossene Außenhülle schützt das pyramidenförmige Gebäude vor dem unerwünschten Eindringen von Wasser etc. von unten, von oben und auch von den Seiten her. Die Geschlossenheit ist so ausgebildet, dass die Außenhülle den Innenraum allseitig vor dem unerwünschten Eindringen von außen von Flüssigkeiten, wie Wasser, oder Wind schützt.

Die Schindelbedeckung besteht aus, vorzugsweise unbehandelten, Schindeln. Eine Schindel kann zur besseren Befestigung an der Außenhülle mindestens eine Durchgangsöffnung aufweisen. Die Form der Durchgangsöffnung, wie einer Bohrung, kann ein zylindrischer Kanal sein. Der Kanalquerschnitt der Durchgangsöffnung muss nicht rund bzw. oval sein. Der Kanalquerschnitt der Durchgangsöffnung kann auch eine Polygonform aufweisen. Die Durchgangsöffnung kann normal durch die Schindel hindurchgeführt sein. Die Durchgangsöffnung kann jedoch auch schräg durch die Schindel hindurchgeführt sein.

Die Seitenflächen sind bevorzugt mehrwandig, d. h. zumindest doppelwandig, ausgeführt. Zwischen ihren mindestens zwei Wänden ist mindestens ein Dämmraum angeordnet. Der Dämmraum kann vorteilhafterweise mit Dämmstoffen ausgekleidet sein. Der Dämmraum kann zumindest teilweise mit Dämmstoffen gefüllt sein. Der Dämmraum kann vollständig mit Dämmstoffen gefüllt sein. Als Dämmstoffe gelangen bevorzugt natürliche Stoffe, wie beispielsweise Schafswolle, zu Anwendung.

Die tragenden Holzbauteile sind Sparren, insbesondere Schrägsparren. Zur sicheren und dauerhaften Befestigung eines Sparrens ist zumindest an einem seiner Enden ein ausgeklinkter Schwalbenschwanz auf Verzug angeordnet. Der ausgeklinkte Schwalbenschwanz dient bevorzugt der Verbindung des Basiskörpers mit dem dem Basiskörper zugewandten Ende eines Sparrens.

Auf der Aufenthaltsplattform sind lehnenlose Bänke angeordnet. Die Bänke sind bevorzugt aus Holz, insbesondere aus einer Lärchenholzart, gefertigt. Bevorzugt sind vier Holzbänke vorhanden. Anstatt der Bänke können auch Stühle, bevorzugt hölzerne Stühle, verwendet werden. Auch eine gemischte Nutzung von Bänken und Stühlen ist natürlich denkbar und möglich. Die Sitz- bzw. Liegegelegenheiten auf der Aufenthaltsplattform sind vorzugsweise parallel zu wenigstens einer Seitenfläche des pyramidenförmiges Gebäudes ausgerichtet.

Eine außermittig befestigte Umlenkrolle für ein Tragemittel ist vorgesehen. Das Tragemittel kann beispielsweise ein Seil sein. Das Seil ist bevorzugt aus einem natürlichen Material. Besonders bevorzugt ist eine Ausführung des Tragemittels als Hanfseil. Die Umlenkrolle mit dem Tragemittel dient zur pendelhaften Aufhängung eines Kristalls. Der Kristall ist bevorzugt ein naturbelassener, unbearbeiteter Bergkristall. Besonders bevorzugt ist ein sechs-flächiger Kristall.

Die Verbindung des ersten und des zweiten Holzbauteiles wird bevorzugt mittels eines Leimes unterstützt. Besonders bevorzugt ist ein Leim auf Holzbasis. Ebenso bevorzugt ist auch ein Leim auf Holzharzbasis. Bei einem Leim auf Holzharzbasis ist ein Leim auf der Basis eines Harzes einer Lärchenholzart besonders bevorzugt. Der Leim unterstützt zumindest die Verbindung des ersten und des zweiten Holzbauteiles in ihrer Haltefähigkeit.

Das pyramidenförmige Gebäude steht bevorzugt auf einem splittbefüllten Bereich. Der splittbefüllte Bereich erleichtert den Wasserabfluß. Der Wasserabfluss ist durch den splittbefüllten Bereich zumindest im Nahbereich des Basiskörpers erleichtert. Der Nahbereich des Basiskörpers umfasst den Bereich unterhalb des Basiskörpers.

Der Ionenstrom bzw. lonenfluss kann mit einem lonenmessgerät, das Bovis-Einheiten misst, bestimmt werden. Der Winkel zwischen der Basisfläche, d. h. der Oberfläche des Basiskörpers, und den der Basisfläche zugewandten Enden der Schrägsparren beträgt bevorzugt 51,8°. Der Winkel in der Spitze (Apixwinkel) sollte bevorzugt 76° betragen. Die Vierseitpyramide hat vorteilhafterweise gleiche Flächen. Die Grundpfosten, vorstehend Pflöcke genannt, stehen bevorzugt direkt auf Kies. Die bei klassischen Steingebäuden bezeichnete Mauerbank ist im Falle der vorliegenden Erfindung aus Baumstämmen gefertigt. Die Fläche der Aufenthaltsplattform beträgt bevorzugt ca. 4,5 mal 4,5 m. Die Höhe beträgt bevorzugt ca. 7,68 m. An der Außenwand liegt vorteilhafterweise nur Splitt, kein Kies, an. Der Abstand der tragenden Innenpfosten für die Aufenthaltsplattform, man könnte in diesem Zusammenhang auch von Holzständern sprechen, beträgt bevorzugt 3,71 m. Die Innenpfosten unterstützen die Schrägsparren. Die Aufenthaltsplattform befindet sich ca. 1/3 der Pyramidenhöhe, bevorzugt der Pyramideninnenhöhe, vom Boden, d. h. von der Oberfläche des Basiskörpers, entfernt. Die Beabstandung kann einige Zentimeter, beispielsweise bis zu 10 cm, nach oben oder nach unten von dem genannten Drittelmaß abweichen. Auch kann die Beabstandung gemessen werden zwischen Boden und Unterseite oder Oberseite der Aufenthaltsplattform. Besonders bevorzugt ist eine Beabstandung der Oberseite der Aufenthaltsplattform vom Boden, d. h. von der Oberfläche des Basiskörpers, im Wert von 1/3 der Pyramidenhöhe, bevorzugt der Pyramideninnenhöhe, plus 2 cm. Die Splitthöhe beträgt bevorzugt ca. 20 cm. In darunter liegenden Schichten sollte Kies, z. B. Rollkies, angeordnet werden. Das Fundament besteht somit aus einer Lage Kies, einem bis zu 20 cm hohen Aufbau Splitt und den umlaufenden Holzbalken, die die aus Holz bestehende Mauerbank bilden. Unter Splitt werden in diesem Zusammenhang wie üblich gebrochene Mineralstoffe, d. h. Steine, deren Form nicht natürlich entstanden ist, sondern die künstlich zerkleinert wurden (Gesteinsbruch), verstanden, und zwar im Unterschied zum Bruchstein natürlicher Herkunft. Kies besteht im Unterschied zu Splitt aus natürlich vorkommenden ungebrochenen Gesteinskörnern.

Es ist denkbar und möglich auf die Abmaße der Pyramide eine Ähnlichkeits-Skalierung anzuwenden.
In absoluten Maßen dargestellte Längen und Winkel sind im Sinne von Relativbeziehungen unter-einander zu verstehen. Den unterschiedlichen Wechselbeziehungen zwischen den Maßen kommt besondere Bedeutung zu. Somit kann eine entsprechend größer oder kleiner skalierte Pyramide mit einem Vielfachen bzw. einem Bruchteil der angegebenen Längen aufgebaut werden. Selbst bei einer Skalierung bleiben die Winkelbeziehungen ähnlich erhalten.

Besonders vorteilhaft ist es, wenn das Holz ca. 3 Tage (abweichend 5 Tage vorher oder nachher) nach Vollmond im Dezember geschlagen worden ist. Das Holz wird vorteilhafterweise schräg nach unten geschlägert. Durch diese Schlägerungsart bleiben die Wipfel der verwendeten Holzart, insbesondere der Lärchenholzart, unbeschädigt am Stamm erhalten. Somit können die Baumwipfel auch die Feuchtigkeit aus dem Holz bis etwa Ende Mai ziehen. Erst danach werden die Baumstämme jeweils auf eine plangemäß verwendbare Länge abgelängt. Danach werden die Stämme jeweils auf die plangemäß verwendbare Dicke zurechtgeschnitten. Das Holz wird vorteilhafterweise so beschnitten, dass kein Splint, d. h. weißer Rand, vorhanden ist. Es wird ausschließlich vollkommen trockenes Holz verwendet. Die Verwendung trockenen Holzes reduziert die im verbauten Zustand noch zu erwartenden bzw. auftretenden Verformungen bzw. den Schwund.

Das gesamte pyramidenförmige Gebäude hat in dem bevorzugten Ausmaß ein Gewicht von ca. 120 t. Die Pyramide steht besonders bevorzugt auf einem positiven Kraftpunkt. Positive Kraftpunkte sind solche Orte auf der Erde, an denen eine positive Wirkung auf die sich auf ihnen befindlichen Personen ausgehen.

Die zuvor ausführlich beschriebene Pyramide kann in ganz bestimmten Arten und Weisen verwendet werden. So ist eine besonders vorteilhafte Verwendung des pyramidenförmigen Gebäudes darin zu sehen, dass sie einen Innenraum bietet, der als zeitweiliger Aufenthaltsraum dienen kann. Der so geschaffene Innenraum dient dazu, Menschen, die sich in ihm befinden, dort eine gewisse Zeit, z. B. eine Stunde, einen Aufenthalt zu bieten, weil sie die Absicht hegen, Linderung und Entspannung von Zuständen des Unwohlseins bzw. der gesundheitlichen Beeinträchtigung zu erfahren. Diese Menschen halten sich hierzu zeitweilig in der Pyramide auf.

### Figurenbeschreibung

Weitere vorteilhafte Eigenschaften und Ausbildungen der Erfindung werden anhand der im Folgenden erläuterten Zeichnungen beschrieben. In diesen Zeichnungen zeigt
- Fig. 1: eine Schrägansicht auf eine erste Ausführungsform eines erfindungsgemäßen pyramidenförmigen Gebäudes;
- Fig. 2: eine Draufsicht von oben auf die Spitze und die Seitenflächen, Schrägsparren und Fenster des pyramidenförmigen Gebäudes;
- Fig. 3: eine Schrägansicht auf tragende Holzbauteile des erfindungsgemäßenpyramidenförmigen Gebäudes aus Fig. 1;
- Fig. 4: eine Schrägansicht auf die tragenden Holzbauteile einer Seitenfläche des erfindungsgemäßen pyramidenförmigen Gebäudes aus Fig. 1; die übrigen Seitenflächen der Übersichtlichkeit halber entfernt;
- Fig. 5: Schrägsicht auf eine Ausführungsform eines hölzernen Sichelzapfens als metallfreier Fremdverbinder;
- Fig. 6: Schrägsicht auf eine Ausführungsform eines hölzernen Schwalbenschwanzes alsmetallfreier Fremdverbinder;
- Fig. 7: Schrägsicht auf zwei Ausführungsformen von metallfreien Längsverbindungen, oben mittels durchgehendem Schwalbenschwanzzapfen, unten mittels Schwalbenschwanz-Blattstoß;
- Fig. 8: Schrägsicht auf eine Ausführungsform einer metallfreien Schrägverbindung mittels einem ausgeklinkten Schwalbenschwanz auf Verzug;
- Fig. 9: Schrägsicht auf eine weitere Ausführungsform einer metallfreien Längsverbindung mittels doppeltem Schwalbenschwanz-Blattstoß;
- Fig. 10: Schrägsicht auf eine Ausführungsform einer metallfreien Flächenverbindung mittels Schwalbenschwänzen mit vorderem auf Gehrung abgesetzten Zinken;
- Fig. 11: Schrägsicht auf eine Ausführungsform einer Holzschraube als metallfreier Fremdverbinder;
- Fig. 12: Schrägsicht auf eine Ausführungsform der von vier Holzständern getragenen Aufenthaltsplattform;
- Fig. 13: Schrägsicht auf eine weitere Ausführungsform der Aufenthaltsplattform mit vier Strömungsaussparungen;
- Fig. 14: Schrägsicht auf das pyramidenförmige Gebäude von Fig. 1, zwei Seitenflächen jedoch entfernt, um die Bänke und das Belüftungssystem sichtbar zu machen;
- Fig. 15: Schrägsicht auf einen Ausschnitt der Schindelbedeckung;
- Fig. 16: Längsschnitt durch eine mit einem Dorn an einem Quersparren befestigte Schindel;
- Fig. 17: Schrägsicht von unten auf eine Ausführungsform der außermittig befestigten Umlenkrolle für ein Hanfseil zur pendelhaften Aufhängung eines sechs-flächigen naturbelassenen unbearbeiteten Bergkristalls;
- Fig. 18: Schrägsicht auf das pyramidenförmige Gebäude von Fig. 1, alle Seitenflächen mit Ausnahme der Schrägsparren jedoch entfernt, das Belüftungssystem mit Lüftungsschlitzen;
- Fig. 19: Schrägsicht auf eine zweite Ausführungsform des pyramidenförmigen Gebäudes ähnlich jener in Fig. 18, die Holzständer bzw. Innenpfosten der Aufenthaltsplattform jedoch in ihrer Höhe zur statischen Unterstützung bis an die Schrägsparren und das Belüftungssystem herangeführt;

In der Folge wird eine erste Ausführungsform eines erfindungsgemäßen pyramidenförmigen Gebäudes beschrieben.

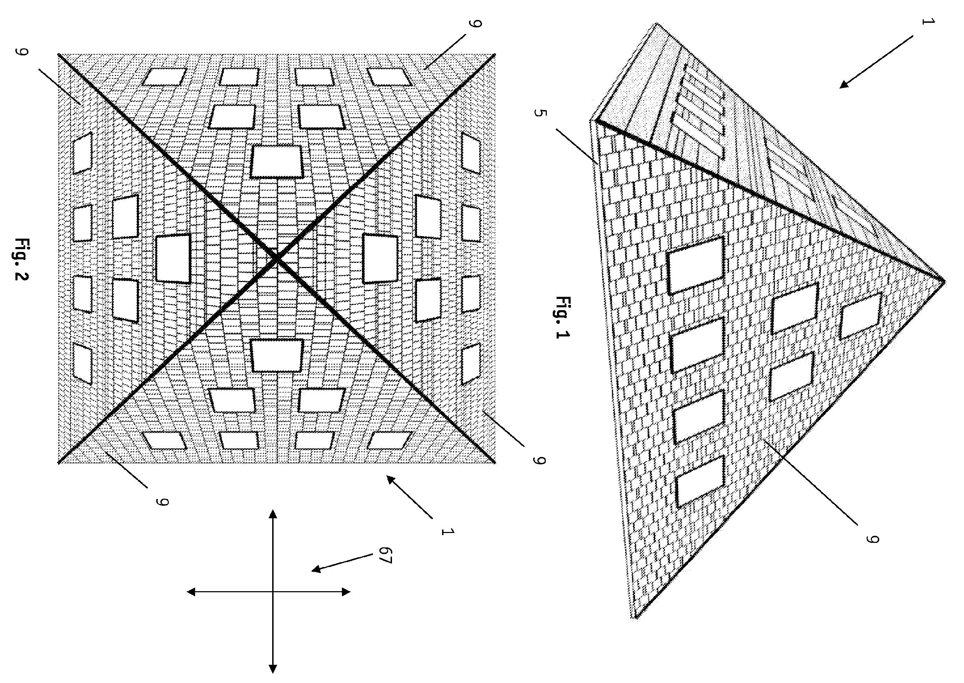

Fig. 1 zeigt eine perspektivische Schrägsicht von der Seite und Fig. 2 eine perspektivische Draufsicht von oben auf die Ausführungsform des pyramidenförmigen Gebäudes 1. Das pyramidenförmige Gebäude 1 weist einen vierseitigen Basiskörper 5 aus einer Lärchenholzart auf. Auch alle anderen Bauteile des pyramidenförmigen Gebäudes 1, insbesondere alle mit dem Bauwerk insbesondere unbeweglich verbundenen Bauteile sind aus Holz, vorzugsweise aus einer Lärchenholzart, gefertigt. Es könnte grundsätzlich auch eine andere Nadelholzart, wie beispielsweise eine Fichten-, Föhren- oder Tannenholzart, sein. Auch Holz einer Laubbaumart, wie beispielsweise eine Buchenholzart, könnte verwendet werden. Auch ist - jedoch weniger bevorzugt - die Verwendung anderer Holzarten, wie Tropenholz- oder Palmenholzarten, denkbar und möglich. Weiterhin ist eine Kombination von verschiedenen Holzarten grundsätzlich denkbar und möglich. Aufgrund ihrer Eigenschaften sind jedoch Lärchenholzarten besonders bevorzugt. Insbesondere ist der homogene Aufbau des pyramidenförmigen Gebäudes 1, d. h. der Aufbau aus einer einzigen Holzart, besonders bevorzugt.

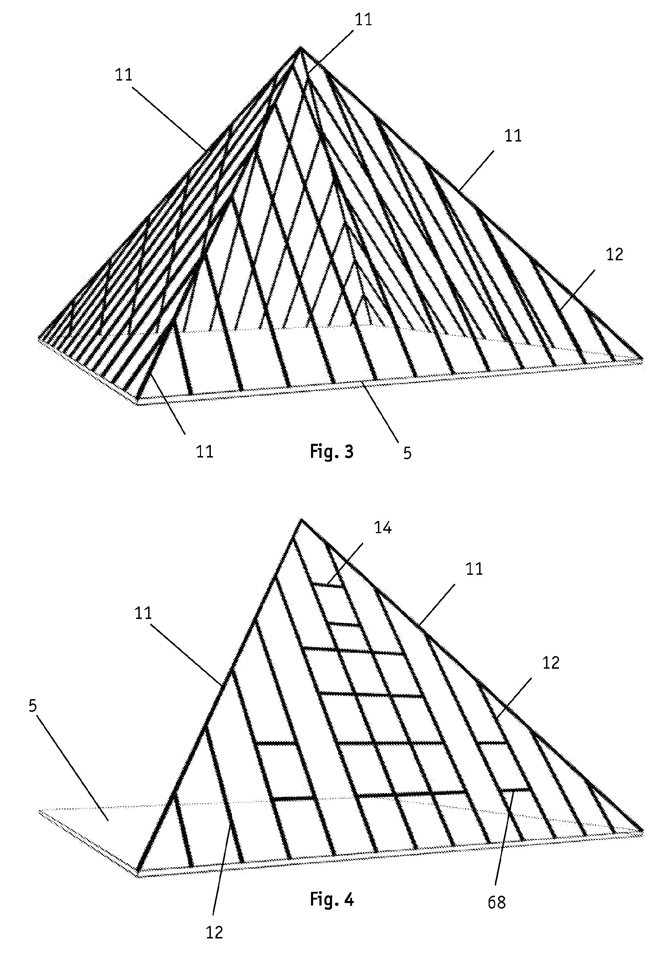

Alle sich über den Basiskörper 5 aufspannenden Seitenflächen 9 sind aus einer einzigen Holzart gefertigt. Die Seitenflächen 9 sind vereinfacht als ähnlich oder gleich geartet bezeichnet, wobei sie aber auch geringe Abweichungen untereinander aufweisen können. In der gezeigten Ausführungsform sind alle vier Seitenflächen 9 aus einer Lärchenholzart gefertigt. Insbesondere sind die tragenden Holzbauteile des pyramidenförmigen Gebäudes 1, wie die Schrägsparren 11 und die Seitenflächensparren 12, aus einer Lärchenholzart gefertigt (vgl. insbesondere die Fig. 3 und Fig. 4).

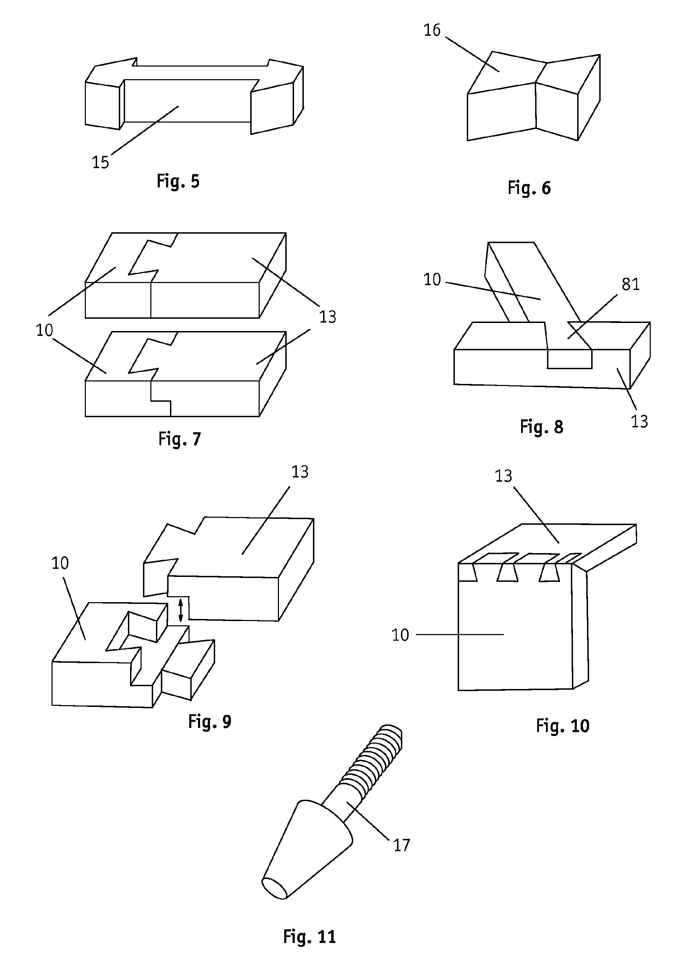

Eine Verbindung eines ersten und eines zweiten Holzbauteiles 10, 13 ist metallfrei zusammengefügt, erfindungsgemäß verwendete Verbindungstechniken sind in den Fig. 5 bis Fig. 11 näher graphisch dargestellt. Die Holzart, insbesondere der tragenden Holzbauteile, ist eine Lärchenholzart, wobei vorzugsweise sämtliche Verbindungen der Holzbauteile 10, 13 durch formschlussbildende, materialgleiche Mittel 15, 16, 17, wie Holzschraube 17, Holzdübel oder Holznagel, realisiert sind. Die Fig. 5, Fig. 6 und Fig. 11 zeigen Beispiele für metallfreie Fremdverbinder als Mittel zur metallfreien Zusammenfügung von Bauteilen der Pyramide. Fig. 5 zeigt eine Ausführungsform eines hölzernen Sichelzapfens 15 zur metallfreien Herstellung einer Längsverbindung. Fig. 6 zeigt eine Ausführungsform eines hölzernen Schwalbenschwanzes 16 als metallfreier Fremdverbinder ebenfalls zur Herstellung einer Längsverbindung. Dieser doppelte Schwalbenschwanz 16 ist hier gerade und symmetrisch ausgebildet. Er könnte jedoch auch schräg bzw. unsymmetrisch und somit zur Herstellung einer Winkelverbindung ausgebildet sein.

Schwalbenschwanzverbindungen sind vor allem wegen ihrer Stabilität, aber auch wegen ihrer Formschönheit, insbesondere für Längs-, Flächen- und Schrägverbindungen, besonders bevorzugt. Die Fig. 7 bis Fig. 10 zeigen Beispiele für geeignete unterschiedliche Ausführungsformen von Schwalbenschwanzverbindungen. Fig. 7 zeigt zwei Beispiele für metallfreie formschlüssige Längsverbindungen, die mit Hilfe von Ausbildungen von Schwalbenschwänzen hergestellt sind. Das obere Beispiel stellt eine metallfreie Längsverbindung mittels durchgehendem Schwalbenschwanzzapfen dar, während das untere Beispiel eine metallfreie Längsverbindung mittels Schwalbenschwanz-Blattstoß zeigt.

Im Pyramidenbau sind insbesondere Schrägverbindungen bzw. Winkelverbindungen erforderlich. Eine Schrägverbindung wird bevorzugt durch einen ausgeklinkten Schwalbenschwanz auf Verzug realisiert. Fig. 8 zeigt eine formschlüssige Schrägverbindung zweier Holzbauteile 10, 13 in Form eines einseitigen Schwalbenschwanzes. Diese Schwalbenschwanzgattung kann insbesondere wie dargestellt als "ausgeklinkt" und "auf Verzug" realisiert werden. Mittels einer solchen Schwalbenschwanz-Ausbildung sind beispielsweise die tragenden Schrägsparren 11 als auch die Seitenflächensparren 12 mit dem Basiskörper 5 des pyramidenförmigen Gebäudes 1 verbindbar bzw. verbunden (siehe Figur 4).

Das Schwalbenschwanz-Prinzip kann auch mehrfach zur Herstellung einer einzelnen Verbindung genutzt werden. Fig. 9 zeigt ein Beispiel einer Längsverbindung zweier Holzbauteile 10, 13 mittels doppeltem Schwalbenschwanz-Stoß.

Weiterhin sind Flächenverbindungen für den Bau diverser Subsysteme des pyramidenförmigen Gebäudes 1 erforderlich. In Fig. 10 ist ein Beispiel einer metallfreien formschlüssigen Flächenverbindung in Form einer Schwalbenschwanz-Zinkung gezeigt, wobei der vordere Zinken auf Gehrung abgesetzt ist. Diese Verbindung ist besonders stabil.

Eine weitere Ausbildung eines hölzernen Fremdverbinders ist beispielsweise eine Holzschraube. Fig. 11 zeigt eine Holzschraube 17, wie sie zur Verbindung von Holzbauteilen des pyramidenförmigen Gebäudes von Fig. 1 Verwendung findet. Mittels solcher Holzschrauben 17 werden bevorzugt insbesondere Innenverkleidungselemente, wie Lärchenholzbretter, mit insbesondere tragenden Holzbauteilen verbunden.

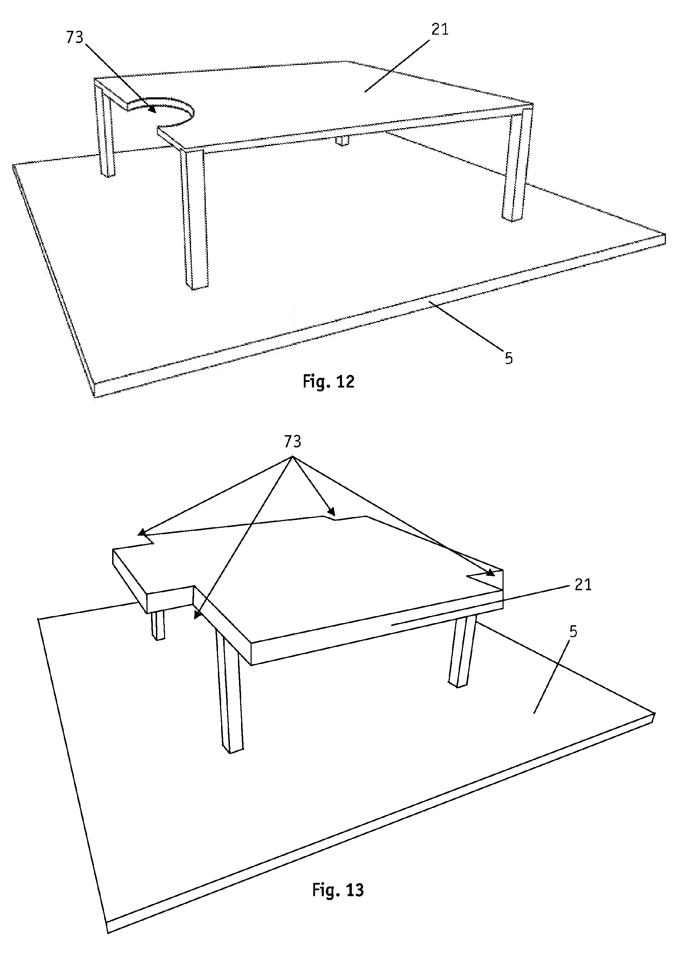

Das pyramidenförmige Gebäude 1 weist eine vom Basiskörper 5 abgehobene, zentrisch zu einem Mittelpunkt des Basiskörpers 5 angeordnete Aufenthaltsplattform 21 auf (Fig. 12, Fig. 13). Der Abstand zwischen einer Oberfläche des Basiskörpers 5 und einer Oberfläche der Aufenthaltsplattform 21 ist vorzugsweise geringfügig größer als ein Drittel einer Gesamthöhe des pyramidenförmigen Gebäudes 1.

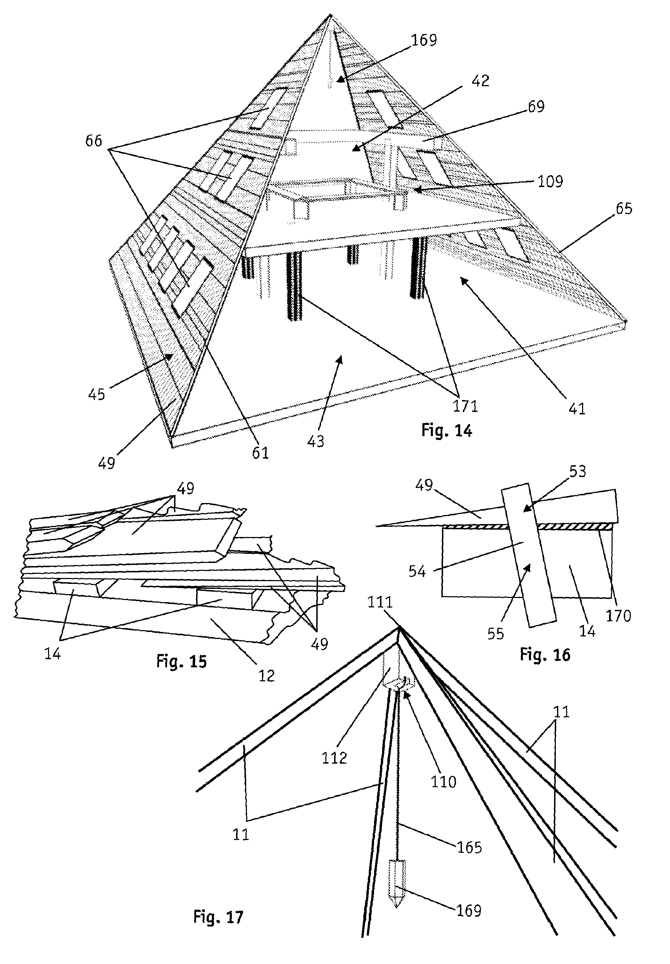

Bei einer in Figur 14 gezeigten, bevorzugten Ausbildung der Aufenthaltsplattform 21 nach Figur 12 oder 13 weist das pyramidenförmige Gebäude 1 nach Figur 1 einen einzigen zusammenhängenden Innenraum 41 auf. Dazu ist die Aufenthaltsplattform 21 zumindest teilweise von den Seitenflächen 9 abgesetzt oder weist Ausschnitte auf, durch die der durch die Aufenthaltsplattform 21 gebildete obere Halbraum 42 der Pyramide mit dem unteren Halbraum 43 kommunizieren kann (Fig. 14). In Fig. 14 ist eine Ausführungsform der Aufenthaltsplattform 21 gezeigt, die von den Seitenflächen 9 abgesetzt ausgebildet ist.

Das pyramidenförmige Gebäude 1 weist eine geschlossene Außenhülle auf. Diese schützt gegen Wind und Wetter, sie ist insbesondere zwar völlig wasserdicht, jedoch gleichzeitig atmungsaktiv ausgebildet. Die geschlossene Außenhülle weist vorzugsweise eine Schindelbedeckung 45 auf.

Die Schindeln 49 sind bevorzugt unbehandelt. Sie weisen, so wie in Figur 16 dargestellt, jeweils mindestens eine Durchgangsöffnung 53 auf, in die ein mit einer Seitenfläche 9 der Pyramide nach Figur 1 verbundener Dorn 54 ragt. Der Dorn 54 kann die Durchgangsöffnung 53 auch völlig durchsetzen und auf der anderen Seite der Schindel 49 ein wenig hervorragen, jedoch nur soviel, dass andere Schindeln 49 durch den Dorn 54 nicht disloziert werden. Fig. 15 zeigt einen Ausschnitt auf die Schindelbedeckung 45 der Pyramide aus Fig. 1. An den Seitenflächensparren 12 einer Seitenfläche 9 sind Quersparren 14 (siehe auch Fig. 4), man könnte auch von einer Querlattung sprechen, metallfrei befestigt, durch die in den Schindelbreiten entsprechenden Abständen Durchgangsbohrungen 55 angeordnet sind. Wie in Fig. 16 dargestellt, ist eine Durchgangsbohrung 55 eines Quersparrens 14 bzw. eine Durchgangsöffnung 53 einer Schindel 49 vorzugsweise schräg zur von ihr durchstoßenen Oberfläche ausgebildet. In die schräge Durchgangsbohrung 55 eines Quersparrens 14 ist der Dorn 54 mit Presssitz eingezwängt. Der Dorn 54 ragt an der Außenseite einer Seitenfläche 9 soweit vor, dass er in eine Durchgangsöffnung 53 einer Schindel 49 ragt. Auf diese Weise sind die Schindeln 49 vorteilhafterweise mit einer Seitenfläche 9 verbunden.

Die Seitenflächen 9 sind zumindest doppelwandig mit einem zwischen ihren mindestens zwei Wänden angeordneten Dämmraum 61 ausgebildet. Der Dämmraum 61 ist vorzugsweise mit Schafswolle 65 ausgefüllt (Fig. 14). Neben der bevorzugten Art der Dämmung, könnte er auch mit anderen natürlichen Materialien, wie beispielsweise gepresstem Stroh oder mit Holzspänen, vorzugsweise Holzspänen einer Lärchenholzart, aufgefüllt sein.

Das pyramidenförmige Gebäude 1 weist symmetrisch angeordnete Fenster 66 in mindestens einer Ebene, vorzugsweise in drei Ebenen, auf. Jedes Fenster 66 ist vorzugsweise doppelwandig ausgebildet. Ein Fensterkasten ist metallfrei mit einer Seitenfläche 9 verbunden. Vorzugsweise ist ein Fensterkasten jeweils sowohl an zwei Seitenflächensparren 12 als auch an zwei Quersparren 68 einer Seitenfläche 9 angebracht (Fig. 4).

Es ist denkbar und möglich, die Pyramide auch ohne jegliches Fenster auszubilden. Auch eine Ausbildung mit beispielsweise vier oder fünf Reihen an Fenstern ist möglich. Die Anzahl an Fensterreihen ist vorteilhaft an die Ausmaße der Pyramide, insbesondere ihre Seitenflächen-Größe, anpassbar. Insbesondere bei einer Pyramide deren Höhe größer oder sogar wesentlich größer als die bevorzugte Höhe von 7,68 Meter ist, können vorteilhaft die Anzahl der Fensterreihen angepasst werden. Die Anzahl der Fensterreihen ist neben der schieren Höhe der Pyramide auch an die unterschiedlich ausbildbare Größe, insbesondere Flächengröße bzw. Fensterhöhe, der Fenster anpassbar. , ausgestattet werden kann. Sollte ein größeres Gebäude erstellt werden, so können somit auch vier oder sogar fünf Reihen oder mehr auf unterschiedlichen Ebenen anzuordnender Fenster 66 platziert werden. Eine besonders abschottende Atmosphäre entsteht in einer Pyramide, wenn keinerlei Fenster vorgesehen sind.

Die Seitenflächen 9 des pyramidenförmigen Gebäudes 1 sind jeweils nach einer der vier Haupthimmelsrichtungen 67 ausgerichtet. Man könnte auch sagen, die Seitenkanten des Basiskörpers 5 sind den Haupthimmelsrichtungen 67 gemäß ausgerichtet. Die Haupthimmelsrichtungen 67, Norden, Süden, Westen und Osten, sind in Fig. 2 durch zwei einander im rechten Winkel schneidende Doppelpfeile angedeutet.

Das Flächenverhältnis der Aufenthaltsplattform 21 zum Basiskörper 5 beträgt mindestens etwa ein Zehntel. Für die gezeigten Ausführungsformen der Aufenthaltsplattform 21 (Fig. 12, Fig. 13 und Fig. 14) ist das Flächenverhältnis jedoch jeweils wesentlich größer. Es besitzt beispielsweise bei der in Fig. 14 dargestellten Ausbildung der Aufenthaltsplattform 21 eine nur aufgrund der Absetzung von den Seitenflächen 9 geringfügig kleinere Fläche als die theoretisch größtmögliche Fläche. Hier sind es etwas weniger als vierundsechzig Quadratmeter, nämlich etwas mehr als zweiundsechzig Quadratmeter.

Eine solche Pyramide benötigt für den Zutritt von Menschen auf die Aufenthaltsplattform 21 einen entsprechenden Zugang, beispielsweise durch eine über eine hölzerne Außentreppe als Aufstiegshilfe erreichbare hölzerne Schiebetür, welche auf der Höhe der Aufenthaltsplattform 21 an einer der Seitenflächen 9 angebracht ist (nicht dargestellt). Bei den in den Fig. 12 und Fig. 13 dargestellten Ausbildungen der Aufenthaltsplattform 21 sind entsprechende Ausschnitte für den Zugang vorgesehen, weshalb hier der Zugang zur Aufenthaltsplattform 21 auch vom unteren Halbraum 43 der Pyramide aus möglich ist. In diesem Fall ist eine entsprechende Zugangsvorrichtung für den unteren Halbraum vorgesehen (nicht dargestellt). Aufgrund der Ausschnitte, wie Strömungsecken und Zugangsöffnungen, sind die Flächen der Aufenthaltsplattform 21 hier wesentlich kleiner ausgebildet.

Der Zugang durch die Außenhülle des Basiskörpers kann von allen Seiten aus, d. h. aus zumindest einer Himmelsrichtung, nach der die Seiten des Basiskörpers ausgerichtet sind, erfolgen. Vorzugsweise erfolgt ein Zugang in den Basiskörper von Süden nach Norden. Diese Ausrichtungsvarianten des Zuganges sind sowohl für oberirdische als auch für unterirdische Ausführungen des Zugangs realisierbar. Eine Pyramide kann mehr als einen Zugang aufweisen. Zugänge können von unterschiedlichen Richtungen aus erfolgen, vorzugsweise jedoch von Süden nach Norden.

In einer alternativen, nicht grafisch dargestellten Ausführungsvariante kann auch ein unterirdischer Zugang, z. B. über einen Tunnel, vorgesehen werden. Die Außenhaut der Pyramide ist in diesem Fall oberirdisch nirgendwo, zumindest nicht durch einen Zugang, durchbrochen. Die Pyramide hätte einen Zugang von der Unterseite. Ein Zugang von der Unterseite schließt die Ausbildung mit Fenstern 66, wie oben beschrieben, in keiner Weise aus. Jedoch ist es denkbar und möglich eine Pyramide mit einem unterirdischen Zugang und ohne jegliche Fenster auszubilden. Die Nutzer der Pyramide würden im Falle der Ausbildung eines unterirdischen Zugangs über ein Tunnelsystem unterirdisch unter die Pyramide gelangen, um anschließend in den einfach zusammenhängenden inneren Aufenthaltsraum der Pyramide zu gelangen. Ein unterirdischer Zugang, d. h. ein Zugang von der Unterseite der Pyramide her, würde an einer geeigneten Stelle in den Basiskörper einzuarbeiten sein.

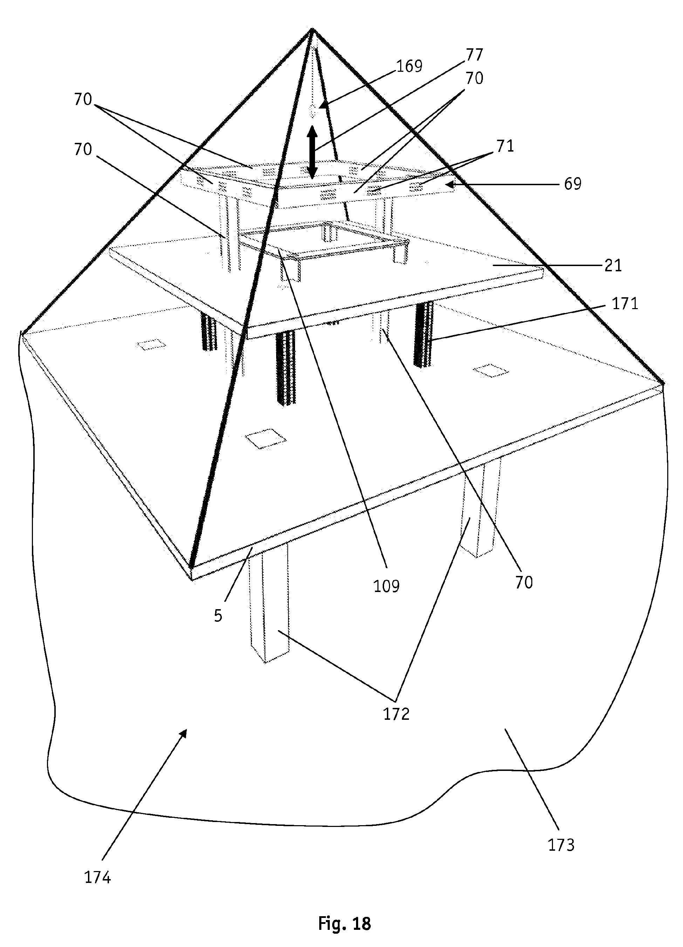

Wie in den Figuren 14 und 18 zu sehen ist, stellt ein Belüftungssystem 69 die Klimatisierung des pyramidenförmigen Gebäudes 1 bereit (insbesondere Fig. 18). Das Belüftungssystem 69 weist hierzu vorzugsweise Lüftungskanäle 70 auf. Eine Aufbereitungsanlage (nicht dargestellt) beschickt zumindest einen der Lüftungskanäle 70 mit konditionierter, beispielsweise vorgewärmter Luft. Die Aufbereitungsanlage könnte die Luft auch ionisieren oder mit Ionen, beispielsweise mit negativen Ionen einer geeigneten Gasspezies anreichern, was aber um die natürliche Wirkung der Pyramide nicht zu beeinflussen weniger bevorzugt ist. Die Aufbereitungsanlage könnte der Luft auch ätherische Duftstoffe bzw. andere Wirkstoffe z.B. in Form von Aerosolen zusetzen. Die Aufbereitungsanlage kann auch beispielsweise verbrauchte oder zu warme Luft über zumindest einen Lüftungskanal 70 des Belüftungssystems 69 absaugen.

Ein Lüftungskanal 70 bzw. ein Abschnitt eines Lüftungskanals 70 weist vorzugsweise Lüftungsschlitze 71 zum Ein- bzw. Ableiten von Luft, gegebenenfalls inklusive von deren Inhaltsstoffen, aus dem Innenraum 41 (siehe Figur 14), insbesondere dem oberen Halbraum 42, auf. Fig. 18 zeigt eine solche Ausbildung des Belüftungssystems 69 mit zwei vertikalen Lüftungskanälen 70, welche beide zum Zu- bzw. Ableiten von Luft zu einem Ring aus vier horizontalen Lüftungskanälen 70, welcher oberhalb der Aufenthaltsplattform 21 angeordnet ist, dienen. Die Lüftungskanäle 70 sind vorzugsweise aus einer Lärchenholzart gefertigt. Sie könnten auch aus einer anderen Holzart, insbesondere Nadelholzart gefertigt sein. Ein vertikaler Lüftungskanal 70 kann unabhängig von zumindest einem weiteren vertikalen Lüftungskanal 70, gesteuert z. B. durch ein hölzernes Klappensystem, als Zuluft- oder als Abluftkanal betrieben werden, während der zumindest eine andere vertikale Lüftungskanal 70 entweder gegensinnig oder gleichsinnig als Zuluft- oder als Abluftkanal betrieben werden kann. Ein horizontaler Lüftungskanal 70 weist sowohl der ihm nächstliegenden Seitenfläche 9 zugewandten Seite als auch von dieser abgewandte Seite Lüftungsschlitze 71 auf. Mit anderen Worten, der obere Lüftungsring weist außen- und innenseitig Lüftungsschlitze 71 auf. Er könnte auch nur innenseitig oder nur außenseitig Lüftungsschlitze aufweisen. Auch die vertikalen Lüftungskanäle könnten Lüftungsschlitze aufweisen.

Das Belüftungssystem 69 könnte auch eine andere Kanalführung oder Kanalkonfiguration aufweisen. Beispielsweise könnte zusätzlich im unteren Halbraum 43 ein Lüftungsring beispielsweise entlang der Seitenkanten des Basiskörpers 5 angeordnet sein. Das Belüftungssystem 69 könnte auch Teile des Basiskörpers 5, insbesondere zumindest einen Hohlraum bzw. einen Kanal im Basiskörper, als Leitungselemente oder Luftspeicherelemente nutzen. Es könnten auch mehr als zwei vertikale Lüftungskanäle vorhanden sein. Ein Lüftungsring könnte auch mehr als viereckig ausgebildet sein, er könnte insbesondere achteckig ausgebildet sein. Bei einer solchen Ausbildung könnte beispielsweise in der Nähe einer Seitenflächenkante jeweils ein weiterer Lüftungskanal 70 in einem Winkel zu den parallel zu den Seitenflächen 9 ausgerichteten Lüftungskanälen 70 angeordnet sein.

Die Aufenthaltsplattform 21 ist vorzugsweise, wie in Fig. 13 dargestellt, kreuzförmig ausgebildet. Die vier Aussparungen der kreuzförmigen Aufenthaltsplattform 21 bilden jeweils an zwei entlang eines Schrägsparrens 11 miteinander verbundenden Seitenflächen 9 eine die Klimatisierung unterstützende Strömungsecke 73. Bei der Ausbildung in Fig. 13 ist eine der Strömungsecken 73 größer als die anderen drei Strömungsecken 73 ausgebildet und zwar so groß, dass sie zusätzlich beispielsweise für einen Treppenaufgang als Aufstiegshilfe auf die Aufenthaltsplattform 21 genutzt werden kann, damit Menschen vom Inneren 43 der Pyramide 1 aus die Aufenthaltsplattform 21 erreichen können.

Durch die beschriebenen Anordnungen des Belüftungssystems 69 mit und ohne Strömungsecken 73 lassen sich gut funktionierende umlauffähige Luftsysteme und Klimatisierungen im Innenraum 41 der Pyramide 1 schaffen, bei denen die verbrauchte Luft effektiv aufgebracht werden kann, während ausreichend frische Luft, im Winter entsprechend beheizt, nachgeliefert wird. Trotz der Klimatisierung hat ein das Gebäude betretender Mensch nicht den Eindruck in einem Frischluftzug zu stehen oder zu sitzen.

Das pyramidenförmige Gebäude 1, vorzugsweise mit Ausnahme eines zumindest weitgehend in seinem Inneren in Luft persistierenden Stromes negativer Ionen 77, ist gänzlich frei von Vorrichtungen, die einen elektrischen Strom oder einen Wasserstrom leiten können. Mit anderen Worten gibt es nirgendwo in der Pyramide elektrische Leiter und auch keine Wasserleitungen.

Die tragenden Holzbauteile sind Sparren, insbesondere Schrägsparren 11, 12. Zur besseren Unterscheidbarkeit wird in dieser Schrift im Hinblick auf einen Sparren, der sich von einer der vier Ecken des Basiskörpers 5 zur Spitze der Pyramide 1 erstreckt, von einem Schrägsparren 11 oder von einem Kantenschrägsparren 11 gesprochen. Während die sonstigen tragenden Schrägsparren einer Seitenfläche 9 als Seitenflächensparren 12 bezeichnet werden. Zumindest die Schrägsparren, d. h. die Kantenschrägsparren 11 und die Seitenflächensparren 12, sind vorzugsweise jeweils zumindest an ihrem einen Ende als ausgeklinkter Schwalbenschwanz auf Verzug 81 ausgebildet, wie in Figur 8 näher dargestellt.

Wie in der Figur 18 dargestellt und auch in Figur 14 zu sehen ist, sind auf der Aufenthaltsplattform 21 sind lehnenlose Bänke 109, insbesondere vier Holzbänke, angeordnet. Die Bänke 109 sind vorzugsweise parallel zu wenigstens einer Seitenfläche 9 des Basiskörpers 5 ausgerichtet. In den Fig. 14 und Fig. 18 ist jede der vier Holzbänke 109 jeweils zu der ihr nächstliegenden Seitenkante des Basiskörpers 5 bzw. zur einer Seitenfläche 9 des pyramidenförmigen Gebäudes 1 angeordnet. Mit anderen Worten bilden die Holzbänke 109 hier eine Sitzgelegenheit entlang einer rechteckigen, vorzugsweise quadratischen Fläche auf der Aufenthaltsplattform 21 aus. Die Bänke 109 können auch eine andere Orientierung zueinander aufweisen. Beispielsweise könnten alle Bänke 109 auch parallel zueinander und parallel zu einer Seitenfläche 9 ausgerichtet sein. Eine bewusste Anordnung der Bänke 109 in Bezug auf eine Seitenfläche gilt als besonders vorteilhaft, insbesondere in Bezug auf das Wohlbefinden des Sitzenden oder Liegenden.

Weiterhin ist eine außermittig befestigte Umlenkrolle 110 für ein Tragemittel 165, wie ein Seil, insbesondere ein Hanfseil, zur pendelhaften Aufhängung eines insbesondere sechs-flächigen Kristalls 169, vorzugsweise eines naturbelassenen, unbearbeiteten Bergkristalls, vorgesehen. Die Umlenkrolle 110 ist in Fig. 17 in einer Schrägsicht von unten auf die Pyramidenspitze 111 dargestellt. Zur besseren Orientierung sind jeweils die obersten Abschnitte der vier in der Pyramidenspitze 111 zusammenlaufenden Schrägsparren 11 ebenfalls dargestellt. In der Pyramidenspitze 111 ist ein in Richtung der vertikalen durch die Pyramidenspitze und den Mittelpunkt des quadratischen Basiskörpers 5 gehende Symmetriezentralachse der Pyramide 1 nach unten ragender Holzbock 112 befestigt. In die Unterseite und eine Seitenfläche dieses Holzbockes 112 ist eine Nut so eingearbeitet, dass die Drehachse der Umlenkrolle 110 außermittig so im Holzbock verankert werden kann, dass eine Laufseite der Umlenkrolle 110 das Seil 165 exakt in die der vertikalen Symmetriezentralachse der Pyramide entsprechende Richtung lenkt.

Ein sich zumindest weitgehend in Luft bewegender Strom negativer Ionen 77 bewegt sich in erhaltender Weise zumindest im Innenraum 41 des pyramidenförmigen Gebäudes 1. Dieser Ionenstrom 77 ist in Fig. 18 als dicker schwarz ausgefüllter Doppelpfeil dargestellt. Realiter persistiert der negative Ionenstrom 77 vorzugsweise in der Richtung von der Pyramidenspitze 111 in Richtung zur Aufenthaltsplattform 21 bzw. zum Basiskörper 5 hin. Der Ionenstrom 77 könnte jedoch auch in die entgegengesetzte Richtung persistieren. Förderlich in diesem Zusammenhang kann das Belüftungssystem 69 angeführt werden.

Im hier beschriebenen Ausführungsbeispiel weist der Basiskörper 5 eine quadratische Grundfläche, mit einer Seitenlänge von zwölf mal zwölf Meter, auf.

Die Höhe des pyramidenförmigen Gebäudes 1 steht in einer durch einen goldenen Schnitt bestimmbaren Relation zur Seitenlänge des Basiskörpers 5. Bei zwölf Metern Seitenlänge beträgt die Höhe vorzugsweise zwischen ca. 7,41 und 7,68 Meter.

Die Verbindung des ersten und des zweiten Holzbauteiles 10, 13 wird mittels eines Leimes 170, vorzugsweise auf Holzbasis oder auf Holzharzbasis, insbesondere auf der Basis eines Harzes einer Lärchenholzart, in ihrer Haltefähigkeit zumindest unterstützt. Insbesondere können die Dorne 54 in den Durchgangsöffnungen 53 der Schindeln 49 bzw. die Schindeln 49 selbst an den Quersparren 14 mit Hilfe eines solchen Leimes 170 anhaftend gemacht sein (siehe Fig. 16).

Es kann ein splittbefüllter Bereich 174, insbesondere für den Wasserabfluss, zumindest unterhalb des Basiskörpers 5 vorgesehen sein. Der Splitt ist vorzugsweise ein Straßensplitt mit einer Korngröße von ca. 6 bis 8 mm. Natürlich können auch andere Materialien und Korngrößen verwendet werden, solange der Wasserabfluss durch das verwendete Material und die verwendete Korngröße gefördert wird.

In einen Untergrund 173 des Basiskörpers 5 eingelassene, insbesondere dort verankerte, Pflöcke 172 sind in der gezeigten Ausführungsform in Fig. 18 ebenfalls vorhanden. Diese dienen insbesondere zur Verankerung des pyramidenförmigen Gebäudes 1 gegen seitliches Abgleiten, beispielsweise in Erdbebengebieten oder auf schrägen Liegenschaften. Die Pflöcke 172 haben hier einen quadratischen Querschnitt. Der Querschnitt könnte auch anders ausgebildet sein. Er könnte beispielsweise rechteckig oder auch rund oder auch oval ausgebildet sein.

Der Aufstellungsort des gezeigten pyramidenförmigen Gebäudes 1 befindet sich an einem positiven Kraftpunkt. Durch das vermehrte Vorkommen von Ionen, insbesondere von negativen Ionen, an einem solchen Ort, kann die Pyramide ihre günstige Wirkung auf die sich in ihr aufhaltenden Menschen in besonderem Maße ausüben. Das vermehrte Vorkommen der Ionen ist durch Vergleichsmessungen mit einem Luftionenmessgerät nachweisbar.

Andere Ausbildungen des erfindungsgemäßen pyramidenförmigen Gebäudes sind denkbar und möglich, ohne den in den Ansprüchen definierten Schutzbereich zu verlassen. Beispielsweise könnten als Tragevorrichtung für die Aufenthaltsplattform 21 anstatt der in den Zeichnungen, insbesondere in den Fig. 14 und Fig. 18. dargestellten vertikalen Holzständer 171 andere Tragevorrichtungen wie beispielsweise geschwungen geformte Tragekonstruktionen, beispielsweise solche, welche aus gebogenen Lärchenholzelementen mittels des erwähnten Leimes 170 zusammengefügt wurden, vorgesehen sein. Weiterhin könnte anstatt einer in einer Seitenfläche 9 angeordneten Schiebetür alternativ ein unterirdischer Zugang durch den Basiskörper 5 der Pyramide 1 vorgesehen sein.

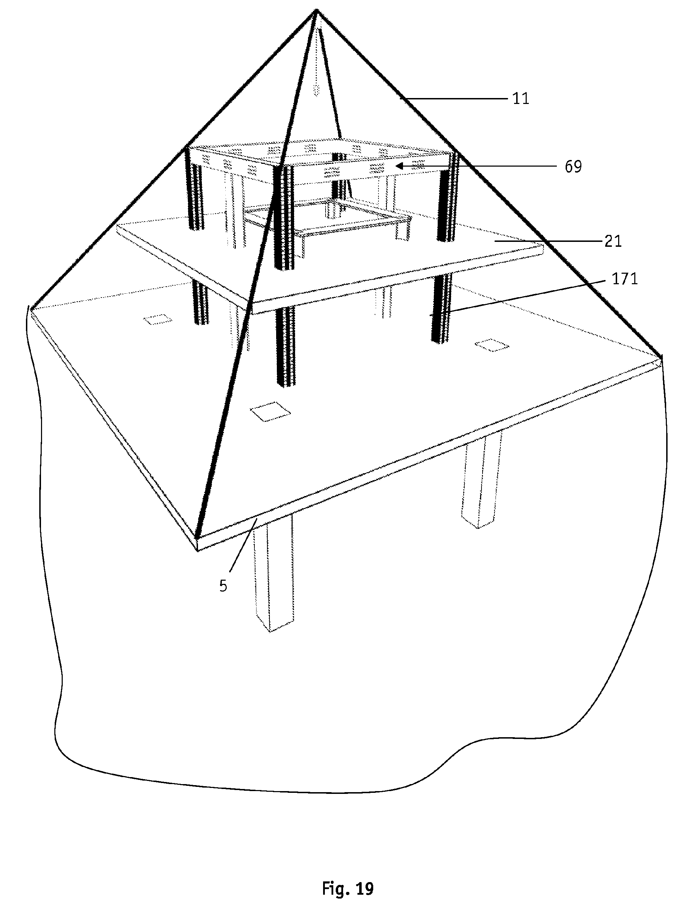

Die Holzständer 171 können in ihrer Höhe bis an die Schrägsparren 11 herangezogen werden. Auf diese Weise können die Holzständer 171 zur statischen Unterstützung der Tragstruktur der Pyramide beitragen Eine solche Ausbildung ist für eine zweite Ausführungsform in Fig. 19 gezeigt. Durch die Verlängerung der Holzständer 171 können die Schrägsparren 11 effektiv in ihrer Tragfunktion unterstützt werden. Die Holzständer 171 tragen dann die Aufenthaltsplattform 21 und unterstützen die Schrägsparren 11. Auch das Belüftungssystem 69 kann sich bei Bedarf, beispielsweise an seinen Ecken, an den bis zu den Schrägsparren hinauf verlängerten Holzständern 171 abstützen. Jeder Holzständer 171 erstreckt sich bei der zweiten Ausführungsform vom Basiskörper 5 aufsteigend die Aufenthaltsplattform 21 tragend bis hinauf zu dem jeweiligen Schrägsparren 11. Es müssen nicht alle Holzständer 171 bis ganz hinauf zu den Schrägsparren geführt sein. Beispielsweise könnten auch nur zwei der vier Holzständer 171 bis zu den Schrägsparren hinauf geführt sein. Bevorzugt ist jedoch die gezeigte Ausführung, bei der jeder der vier Holzständer 171 bis zu jeweils einem Schrägsparren aufsteigt. Die Aufenthaltsplattform könnte natürlich auch von mehr als vier Holzständern 171 getragen sein. Weitere Holzständer 171 könnten zumindest teilweise die in Fig. 19 nicht dargestellten Seitenflächen stützen. Die zusätzlichen Holzständer 171 (nicht dargestellt) könnten jedoch auch nur die Aufenthaltsplattform 21 tragen. Vorteilhaft ist die Anordnung der Holzständer 171 an den Rändern, z. B. an den Ecken, der Aufenthaltsplattform. Bei einer solchen verlängerten Ausbildung der Holzständer 171 kann man auch, wie am Ende der Erfindungsbeschreibung oben angedeutet, von Innenpfosten sprechen. Die Innenpfosten 171 unterstützen die Tragstruktur. Die Innenpfosten 171 sind Teil der Tragsstruktur der Pyramide. Die Innenpfosten 171 unterstützen die Schrägsparren. Die Innenpfosten 171 können das Belüftungssystem 69 unterstützen. Jeweils zwei der Innenposten 171 können zur weiteren Verfestigung der Struktur an ihren oberen Vertikalenden durch Querpfosten miteinander verbunden sein (nicht dargestellt). Die Querposten können sich somit jeweils entlang der Seitenwände erstrecken. Zwei Querpfosten können sich jedoch auch jeweils diagonal von einem Schrägsparren zu dem diesem diagonal gegenüberliegenden Schrägsparren erstrecken.

Weiterhin kann das pyramidenförmige Gebäude 1 auch zumindest eine andere, gegebenenfalls zusätzliche, Aufstiegshilfe als die oben erwähnten Treppen aufweisen. Beispielsweise kann eine barrierefreie Aufstiegshilfe wie eine schräge Rampe vorhanden sein. Insbesondere für querschnittgelähmte oder andere beeinträchtigte bzw. schwer transportierbare Personen kann auch eine Aufzugsvorrichtung vorhanden sein. Eine solche ist vorzugsweise aus Holz mit hölzernen Flaschenzügen mit Naturseilen, wie Hanfseilen, ausgebildet.

Weiterhin kann die Pyramide zum Aufbau eines "Pyramidendorfes", beispielsweise mit ca. drei bis fünf Pyramiden bzw. zum Aufbau mehrerer solcher "Pyramidendörfer", insbesondere auch mit einer jeweils variablen Anzahl von Einzelpyramiden, Verwendung finden. Man könnte ein Pyramidendorf auch als Pyramidencluster bezeichnen. Zumindest zwei Einzelpyramiden eines Pyramidenclusters können vorzugsweise unterirdisch durch ein System von Gängen bzw. Kanälen miteinander verbunden sein. In den Gang- bzw. Kanalsysteme kann bevorzugt die Infrastruktur, beispielsweise für die Belüftungssysteme, insbesondere für die Luftkonditioniersysteme, enthalten sein.

### Liste der Bezugszeichen:

- 1: pyramidenförmiges Gebäude
- 5: Basiskörper
- 9: Seitenfläche
- 10: erstes Holzbauteil
- 11: Schrägsparren
- 12: Seitenflächensparren
- 13: zweites Holzbauteil
- 14: Quersparren
- 15: Sichelzapfen
- 16: doppelter Schwalbenschwanz
- 17: Holzschraube
- 21: Aufenthaltsplattform
- 41: Innenraum
- 42: oberer Halbraum
- 43: unterer Halbraum
- 45: Schindelbedeckung
- 49: Schindel
- 53: Durchgangsöffnung
- 54: Dorn
- 55: Durchgangsbohrung

- 61: Dämmraum
- 65: Schafswolle
- 66: Fenster
- 67: Haupthimmelsrichtungen
- 68: Quersparren
- 69: Belüftungssystem
- 70: Lüftungskanal
- 71: Lüftungsschlitz
- 73: Strömungsecke
- 77: Strom negativer Ionen
- 81: ausgeklinkter Schwalbenschwanz auf Verzug
- 82: Bank
- 78: Umlenkrolle
- 79: Pyramidenspitze
- 80: Holzbock
- 81: Tragemittel
- 82: Kristall
- 83: Leim
- 84: Holzständer
- 85: Pflock
- 86: Untergrund
- 87: splittbefüllter Bereich

## Patentansprüche

1. Pyramidenförmiges Gebäude (1), mit einem vierseitigen Basiskörper (5) aus Holz, wie Nadelholz,
**dadurch gekennzeichnet, dass**
alle sich über den Basiskörper (5) aufspannenden Seitenflächen (9) aus einer einzigen Holzart so gefertigt sind,
dass eine Verbindung eines ersten und eines zweiten Holzbauteiles (10, 13) metallfrei zusammengefügt ist.

2. Pyramidenförmiges Gebäude (1) nach Anspruch 1,
**dadurch gekennzeichnet, dass**
das Holz, insbesondere der tragenden Holzbauteile, eine Lärchenholzart ist,
wobei vorzugsweise sämtliche Verbindungen der Holzbauteile (10, 13) durch formschlussbildende, materialgleiche Mittel (15, 16, 17), wie Holzschraube (17), Holzdübel oder Holznagel, realisiert sind.

3. Pyramidenförmiges Gebäude (1) nach Anspruch 1 oder 2,
**dadurch gekennzeichnet, dass**
das pyramidenförmige Gebäude (1) eine vom Basiskörper (5) abgehobene, zentrisch zu einem Mittelpunkt des Basiskörpers (5) angeordnete Aufenthaltsplattform (21) aufweist,
wobei vorzugsweise der Abstand zwischen einer Oberfläche des Basiskörpers (5) und einer Oberfläche der Aufenthaltsplattform (21) geringfügig größer als ein Drittel einer Gesamthöhe des pyramidenförmigen Gebäudes (1) ist.

4. Pyramidenförmiges Gebäude (1) nach einem der Ansprüche 1 bis 3,
**dadurch gekennzeichnet, dass**
symmetrisch angeordnete Fenster (66) in mindestens einer Ebene, vorzugsweise in drei Ebenen, vorhanden sind,
wobei jedes Fenster (66) doppelwandig ausgebildet ist.

5. Pyramidenförmiges Gebäude (1) nach einem der Ansprüche 1 bis 4,
**dadurch gekennzeichnet, dass**
die Seitenflächen (9) jeweils nach einer der vier Haupthimmelsrichtungen (67) ausgerichtet sind.

6. Pyramidenförmiges Gebäude (1) nach einem der Ansprüche 1 bis 5,
**dadurch gekennzeichnet, dass**
das Flächenverhältnis der Aufenthaltsplattform (21) zum Basiskörper (5) mindestens etwa ein Zehntel beträgt.

7. Pyramidenförmiges Gebäude (1) nach einem der Ansprüche 1 bis 6,
**dadurch gekennzeichnet, dass**
ein Belüftungssystem (69) die Klimatisierung des pyramidenförmigen Gebäudes (1) bereitstellt.

8. Pyramidenförmiges Gebäude (1) nach einem der Ansprüche 1 bis 7,
**dadurch gekennzeichnet, dass**
die Aufenthaltsplattform (21) kreuzförmig ausgebildet ist,
wobei die vier Aussparungen der kreuzförmigen Aufenthaltsplattform (21) jeweils an zwei entlang eines Schrägsparrens (11) miteinander verbundenen Seitenflächen (9) eine die Klimatisierung unterstützende Strömungsecke (73) bilden.

9. Pyramidenförmiges Gebäude (1) nach einem der Ansprüche 1 bis 8,
**dadurch gekennzeichnet, dass**
das pyramidenförmige Gebäude (1), vorzugsweise mit Ausnahme eines zumindest weitgehend in seinem Inneren in Luft persistierenden Stromes von Ionen (77), gänzlich frei von Vorrichtungen ist, die einen elektrischen Strom oder einen Wasserstrom leiten können.

10. Pyramidenförmiges Gebäude (1) nach einem der Ansprüche 1 bis 9,
**dadurch gekennzeichnet, dass**
ein sich zumindest weitgehend in Luft bewegender Strom von Ionen (77), insbesondere mit einem größeren Anteil an negativen als an positiven Ionen, beispielsweise im Verhältnis 80:20, zumindest im Innenraum (41) des pyramidenförmigen Gebäudes (1) persistiert.

11. Pyramidenförmiges Gebäude (1) nach einem der vorstehenden Ansprüche,
**dadurch gekennzeichnet, dass**
der Basiskörper (5) eine quadratische Grundfläche, vorzugsweise mit einer Seitenlänge von zwölf mal zwölf Metern, aufweist.

12. Pyramidenförmiges Gebäude (1) nach einem der vorstehenden Ansprüche,
**dadurch gekennzeichnet, dass**
eine Höhe des pyramidenförmigen Gebäudes (1) in einer durch einen goldenen Schnitt bestimmbaren Relation zur Seitenlänge des Basiskörpers (5) steht,
insbesondere dass bei zwölf Metern Seitenlänge die Höhe 7,68 Meter beträgt.

13. Pyramidenförmiges Gebäude (1) nach einem der vorstehenden Ansprüche,
**dadurch gekennzeichnet, dass**
das pyramidenförmige Gebäude (1) zumindest einen Pflock (172) umfasst, der ein in einem Untergrund (173) des Basiskörpers (5) eingelassener, insbesondere dort verankerter, Pflock (172) insbesondere zur Verankerung des pyramidenförmigen Gebäudes (1) gegen seitliches Abgleiten, beispielsweise in Erdbebengebieten oder auf schrägen Liegenschaften, ist.

14. Pyramidenförmiges Gebäude (1) nach einem der vorstehenden Ansprüche,
**dadurch gekennzeichnet, dass**
sich ein Aufstellungsort des pyramidenförmigen Gebäudes (1) an einem positiven Kraftpunkt befindet.

15. Verwendung eines pyramidenförmigen Gebäudes (1) nach einem der vorstehenden Ansprüche,
**dadurch gekennzeichnet, dass**
das Gebäude (1) zum zeitweiligen Aufenthalt von sich regenerierenden Menschen genutzt wird.
